(19) Europäisches Patentamt
European Patent Office
Office européen des brevets

(11) Veröffentlichungsnummer: **0 295 553 B1**

(12) **EUROPÄISCHE PATENTSCHRIFT**

(45) Veröffentlichungstag der Patentschrift: **21.08.91**

(51) Int. Cl.$^5$: **C07C 69/593**, C07C 69/54, C07C 67/317, C07B 47/00

(21) Anmeldenummer: **88109092.2**

(22) Anmeldetag: **08.06.88**

(54) **Verfahren zur Herstelung von 1,1-disubstituierten Ethylenverbindungen.**

(30) Priorität: **13.06.87 DE 3719873**

(43) Veröffentlichungstag der Anmeldung:
**21.12.88 Patentblatt 88/51**

(45) Bekanntmachung des Hinweises auf die Patenterteilung:
**21.08.91 Patentblatt 91/34**

(84) Benannte Vertragsstaaten:
**BE CH DE FR GB LI NL**

(56) Entgegenhaltungen:
**EP-A- 0 033 881**
**DE-A- 3 010 968**
**GB-A- 1 586 805**
**US-A- 3 928 458**

(73) Patentinhaber: **BASF Aktiengesellschaft**
**Carl-Bosch-Strasse 38**
**W-6700 Ludwigshafen(DE)**

(72) Erfinder: **Merger, Franz, Dr.**
**Max-Slevogt-Strasse 25**
**W-6710 Frankenthal(DE)**
Erfinder: **Liebe, Joerg, Dr.**
**Hans-Purrmann-Strasse 10 b**
**W-6710 Frankenthal(DE)**
Erfinder: **Bertleff, Werner, Dr.**
**Neuzenlache 3**
**W-6806 Viernheim(DE)**

EP 0 295 553 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues und verbessertes Verfahren zur Herstellung von 1,1-disubstituierten Ethylenverbindungen.

Die Herstellung von 2-substituierten Acrylsäureestern aus 2-Formylcarbonsäureestern durch Hydrierung der Formylgruppe zur Hydroxygruppe und anschließende Wasserabspaltung ist bekannt (z.B. GB 1 586 805 und P.E. Pfeffer et.al., J. Org. Chem. 37, 1256 (1972)). Analog lassen sich aus 2-Formylnitrilen oder α-Formylketonen die entsprechenden ungesättigten Verbindungen herstellen. Hierbei sind jedoch zwei Reaktionsschritte erforderlich, die Ausbeuten sind nicht sehr hoch.

Itaconsäurediester

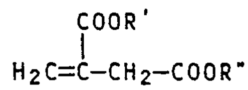

werden technisch aus Itaconsäure und Alkoholen in Gegenwart von Säuren durch klassische Veresterung hergestellt.

Itaconsäure wird gegenwärtig biochemisch durch Fermentation von Kohlenhydraten (Melasse) hergestellt. Das biochemische Syntheseverfahren ist technisch recht aufwendig, führt zu geringen Raum-Zeit-Ausbeuten und ist daher mit hohen Kosten verbunden. Es hat jedoch bislang noch den Vorzug vor einer Reihe chemischer Synthesen wie trockener Destillation von Citronensäure, Oxidation von Isopren oder Mesityloxid zu Citraconsäure mit anschließender Isomerisierung, Carboxylierung von Acetylenderivaten wie z.B. Propargylchlorid oder Butinsäureestern oder Kondensation von Bernsteinsäureestern oder Bernsteinsäureanhydrid mit Formaldehyd zu Citraconsäure mit anschließender Isomerisierung, die nicht wirtschaftlich durchführbar sind, da sie z.B. viele Stufen beinhalten, unbefriedigende Ausbeuten ergeben oder von schwer zugänglichen Ausgangsstoffen ausgehen. (Vgl. Kirk-Cthmer, Vol. 13, Seite 865-870; B.E. Tate, Itaconic Acid, Itaconic Esters and Related Compounds, in E.C. Leonard, Vinyl and Diene Monomers. Part 1, Wiley Interscience, New York 1970, Seiten 205-261).

Der Erfindung lag daher die Aufgabe zugrunde, ein neues und verbessertes Verfahren zur Herstellung von 1,1-disubstituierten Ethylenverbindungen aus Formylverbindungen zu finden und den Nachteilen der bekannten Verfahren abzuhelfen.

Demgemäß wurde ein Verfahren zur Herstellung von 1,1-disubstituierten Ethylenverbindungen der allgemeinen Formel I

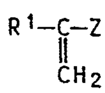

(I),

in der

- Z $COOR^2$, CN oder $COR^3$,
- $R^1$ einen aliphatischen, cycloaliphatischen, araliphatischen, aromatischen oder heterocyclischen Rest, der gegebenenfalls noch durch unter den Reaktionsbedingungen inerte funktionelle Gruppen weitersubstituiert sein kann,
- $R^2$ einen aliphatischen, cycloaliphatischen oder araliphatischen Rest mit 1 bis 15 Kohlenstoffatomen und
- $R^3$ einen aliphatischen, cycloaliphatischen oder araliphatischen Rest mit 1 bis 15 Kohlenstoffatomen, der auch durch unter den Reaktionsbedingungen inerte Gruppen substituiert sein kann,

bedeuten, wobei $R^1$ mit $R^2$ bzw. $R^1$ mit $R^3$ aber auch eine Alkylenkette mit 2 bis 10 Kohlenstoffatomen, die durch unter den Reaktionsbedingungen inerte Gruppen substituiert sein kann, bilden kann, aus Formylverbindungen der allgemeinen Formel II

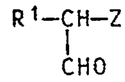 (II),

in der Z, $R^1$, $R^2$ und $R^3$ die oben genannten Bedeutungen besitzen, gefunden, welches dadurch gekennzeichnet, daß man die Umsetzung in Gegenwart eines sekundären Amins, einer Protonensäure und von Formaldehyd oder Paraformaldehyd und

a) einem $C_1$-$C_{12}$-Alkanol oder

b) einem Gemisch aus einem $C_1$-$C_{12}$-Alkanol und Wasser oder

c) mit Wasser

bei Temperaturen von 0 bis 200 °C vornimmt.

Die Umsetzung kann z.B. für den Fall der Verwendung von 2-Formylpropionsäuremethylester durch folgendes Reaktionsschema wiedergegeben werden:

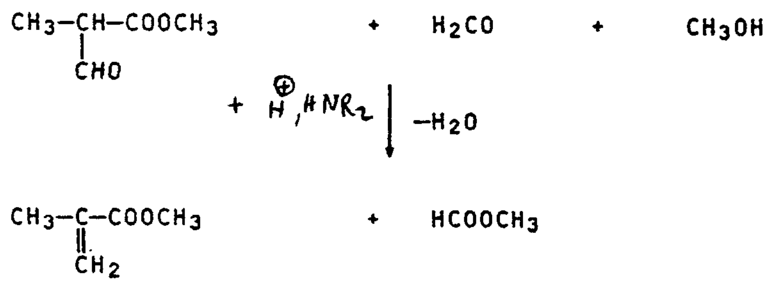


oder für den Fall der Verwendung von 2-Formylbernsteinsäuredimethylester durch das folgende Reaktionsschema wiedergegeben werden:

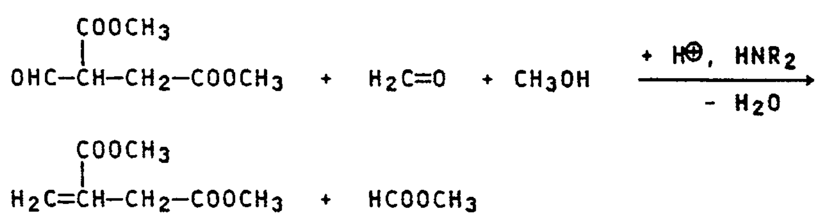


Die für die Umsetzung benötigten Formylverbindungen II können auf bekannten Wegen erhalten werden, z.B. durch eine Esterkondensation von Carbonsäureestern, von Carbonsäurenitrilen oder von Ketonen mit Ameisensäurealkylestern oder bevorzugt durch Hydroformylierung von $\alpha,\beta$-ungesättigten Cabonsäureestern, Carbonsäurenitrilen oder Ketonen.

Bevorzugt verwendet man 2-Formylcarbonsäureester, 2-Formylcarbonsäurenitrile oder 2-Formylketone der allgemeinen Formel II mit Z = $COOR^2$, CN oder $COR^3$, worin $R^1$ für einen Alkylrest mit 1 bis 12, insbesondere 1 bis 8 Kohlenstoffatomen, einen Alkenylrest mit 2 bis 12 Kohlenstoffatomen, einen Oxaalkylrest mit 1 bis 12 Kohlenstoffatomen und 1 bis 5 Sauerstoffatomen, einen Cycloalkylrest mit 5 bis 8 Kohlenstoffatomen, einen Aralkylrest mit 7 bis 12 Kohlenstoffatomen, einen Arylrest mit 6 bis 12 Kohlenstoffatomen oder einen hetercyclischen Rest mit 2 bis 6 Kohlenstoffatomen und 1 bis 3 Stickstoff-, Sauerstoff- oder Schwefelatomen, $R^2$ für einen Alkylrest mit 1 bis 8 Kohlenstoffatomen, einen Cycloalkylreste mit 5 bis 8 Kohlenstoffatomen oder einen Aralkylrest mit 7 bis 12 Kohlenstoffatomen, $R^3$ für einen Alkylrest mit 1 bis 12, insbesondere 1 bis 8 Kohlenstoffatomen, einen Alkenylrest mit 2 bis 12 Kohlenstoffatomen, einen Oxaalkylrest mit 1 bis 12 Kohlenstoffatomen und 1 bis 5 Sauerstoffatomen, einen Cycloalkylrest mit 5 bis 8 Kohlenstoffatomen, einen Aralkylrest mit 7 bis 12 Kohlenstoffatomen, einen Arylrest mit 6 bis 12 Kohlenstoffatomen steht, wobei $R^1$ mit $R^2$ bzw. $R^1$ mit $R^3$ aber auch eine Alkylenkette mit 2 bis 8 Kohlenstoffatomen, die durch unter den Reaktionsbedingungen inerte Gruppen substituiert sein kann, bilden

kann. Die vorgenannten Reste können durch zusätzliche, unter den Reaktionsbedingungen inerte Gruppen, insbesondere Alkyl-, Alkoxi-, Halogen-, Ester-, Nitril- oder tert.-Aminogruppen weiter substituiert sein. Besonders bevorzugte Ausgangsstoffe sind 2-Formylpropionsäurealkylester, 2-Formylbuttersäurealkylester und 2-Formylbernsteinsäurealkylester.

Beispielsweise seien folgende Ausgangsstoffe genannt:
2-Formylpropionsäuremethylester, 2-Formylpropionsäureethylester, 2-Formylbuttersäuremethylester, 2-Formylbuttersäureethylester, 2-Formylbernsteinsäuredimethylester, -diethylester, -dipropylester, -diisopropylester, -dibutylester, dihexylester, -dioctylester, -didecylester, -diallylester, -di(2-methoxyethyl)-ester, -dicyclohexylester, -ethylmethylester, -butylmethylester oder -dibenzylester.

Formaldehyd kann in wäßriger Form, bevorzugt in 20 bis 60 prozentiger wäßriger Lösung, in wasserarmer Form, z.B. gelöst in einem Gemisch aus Wasser, $C_1$-$C_8$-Alkanol und gegebenenfalls Lösungsmittel, oder in wasserfreier Form, z.B. gasförmig, gelöst in einem $C_1$-$C_8$-Alkanol gegebenenfalls in einem Lösungsmittel, oder als Paraformaldehyd eingesetzt werden. Das Molverhältnis zwischen Ausgangsstoff II und Formaldehyd liegt zweckmäßig bei 0,5 bis 2,0:1, Molverhältnisse außerhalb dieses Bereiches sind aber auch möglich.

Als Alkohole eignen sich $C_1$-$C_{12}$-Alkanole, bevorzugt $C_1$-$C_8$-Alkanole, besonders bevorzugt Methanol. Auch mehrwertige Alkohole wie z.B. Glykol oder Glycerin sind einsetzbar. Das Molverhältnis zwischen Ausgangsstoff II und dem Alkanol liegt im allgemeinen bei 1:1 bis 1:50, bevorzugt bei 1:2 bis 1:25, insbesondere bei 1:2 bis 1:15. Molverhältnisse außerhalb dieses Bereiches sind aber auch möglich.

Die Umsetzung verläuft überraschenderweise unter so milden Bedingungen, daß bei Einsatz von Alkanolen, die nicht mit dem Esteralkohol $R^2$-OH übereinstimmen, Umesterungen vermieden werden können.

Anstelle der Alkohole können auch entsprechende Mengen Wasser verwendet werden oder Gemische von Alkoholen und Wasser, insbesondere wasserlöslichen Alkoholen mit Wasser.

Gemäß einer bevorzugten Ausführungsform des erfindungsgemäßen Verfahrens wird das für die Umsetzung benötigte Alkanol oder Wasser im Überschuß eingesetzt und dient dann gleichzeitig als Lösungsmittel. Es können aber auch andere Lösungsmittel bei der Reaktion eingesetzt werden. Geeignet sind z.B. cyclische Ether wie Tetrahydrofuran oder Dioxan.

Als Protonensäure können anorganische oder organische Säuren verwendet werden; anstelle von einbasigen Säuren können auch äquivalente Mengen mehrbasiger Säuren eingesetzt werden. Auch saure Ionenaustauscherharze sind geeignet. Folgende Säuren können beispielsweise eingesetzt werden: aliphatische Monocarbonsäuren mit 1 bis 10 Kohlenstoffatomen wie z.B. Essigsäure, Propionsäure, Buttersäure, Valeriansäure, Hexan-, Heptan-, Octan-, Decansäure; aromatische Carbonsäuren wie z.B. Benzoesäure; saure Ionenaustauscher mit Carboxygruppen oder Sulfonsäuregruppen; aliphatische und aromatische Sulfonsäuren wie z.B. Methansulfonsäure, Benzolsulfonsäure oder p-Toluolsulfonsäure; aliphatische Dicarbonsäuren mit 2 bis 6 Kohlenstoffatomen wie z.B. Oxalsäure, Bernsteinsäure, Adipinsäure oder anorganische Säuren wie z.B. Schwefelsäure, Phosphorsäure, Salzsäure. Bevorzugt werden aliphatische Monocarbonsäuren mit 2 bis 5 Kohlenstoffatomen, besonders bevorzugt Essigsäure, eingesetzt.

Das Molverhältnis von Ausgangsstoff II zur Protonensäure kann zwischen 1:0,005 und 1:1,5, bevorzugt zwischen 1:0,01 und 1:0,5 und besonders bevorzugt zwischen 1:0,01 und 1:0,1 liegen.

Als Amine kommen die für Mannich-Reaktionen üblicherweise verwendeten sekundären Amine in Betracht. Beispielsweise können Amine der Formel III

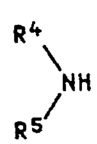

(III)

verwendet werden, in der $R^4$ und $R^5$ gleich oder verschieden sein können und $C_1$-$C_{10}$-Alkylreste, die auch durch inerte Gruppen wie $C_1$- bis $C_4$-Alkoxygruppen oder Hydroxygruppen substituiert sein können oder $C_5$-$C_6$-Cycloalkylreste bedeuten. Bevorzugt werden niedermolekulare Amine, insbesondere $C_2$-$C_{10}$-Dialkylamine wie Dimethyl-, Diethyl-, Dipropyl-, Dibutylamin oder Diethanolamin verwendet, wobei Dimethylamin besonders bevorzugt ist.

Das Molverhältnis vom Ausgangsstoff II zum Amin liegt in der Regel zwischen 1:0,005 und 1:1,5, bevorzugt zwischen 1:0,01 und 1:0,5 und besonders bevorzugt zwischen 1:0,01 und 1:0,1.

Die Reaktion kann bei einer Temperatur von 0 bis 200 °C, bevorzugt 30 bis 150 °C, insbesondere 40

bis 100 °C, drucklos, unter Druck oder unter vermindertem Druck durchgeführt werden.

Die Durchführung der Reaktion kann zweckmäßig in der Weise erfolgen, daß man das Alkanol, ggf. das Lösungsmittel, die Protonensäure und das Amin mischt und den Ausgangsstoff II und Formaldehyd, gasförmig, gelöst in Wasser, gelöst im jeweils eingesetzten Alkanol oder ggf. gelöst in einem Lösungsmittel oder als Paraformaldehyd innerhalb von 0,01 bis 10 Stunden zufügt. Man erhitzt auf Reaktionstemperatur, rührt noch 1 bis 100 Stunden bei Reaktionstemperatur und arbeitet anschließend auf. Man kann auch das Gemisch aus Lösungsmittel. Alkanol, gegebenenfalls Lösungsmittel, Protonensäure und Amin auf Reaktionstemperatur bringen und Formaldehyd und den Ausgangsstoff II bei dieser Temperatur zugeben oder auch alle Komponenten bei tiefer Temperatur (z.B. 0 bis 10 °C) miteinander mischen und anschließend auf die Reaktionstempeatur bringen. Die für die jeweils eingesetzten Ausgangsstoffe und die jeweilige Ansatzgröße optimalen Bedingungen können durch einfache Vorversuche ermittelt werden.

Die Aufarbeitung geschieht auf üblichem Wege, z.B. durch Destillation und/oder Kristallisation. Falls erforderlich, können basische Nebenprodukte durch eine Extraktion mit anorganischen Säuren, z.B. mit wäßriger Schwefelsäure, entfernt werden.

Als Reaktionsprodukte erhält man in sehr guten Ausbeuten die gewünschten 1,1-disubstituierten Ethylenverbindungen und als verwertbares Nebenprodukt die Ameisensäureester des jeweils verwendeten Alkanols, also z.B. bei Einsatz von Methanol Methylformiat. Die Ameisensäurealkylester können als solche verwertet werden oder durch Hydrolyse in rückführbares Alkanol und Ameisensäure gespalten werden.

Ein besonderer Vorteil des erfindungsgemäßen Verfahrens ist die Reinheit der erhaltenen, 1,1-disubstituierten Ethylenverbindungen, Isomerisierungen der Doppelbindungen treten nicht oder nicht nennenswert auf. So sind z.B. bei der Herstellung von Itaconsäuredimethylester weder Citracon- noch Mesaconsäuredimethylester nachweisbar.

Die nach dem Verfahren herstellbaren 1,1-disubstituierten Ethylenverbindungen wie 2-substituierten Acrylsäurealkylester, Acrylnitril bzw. α-Methylenketone sind wertvolle Ausgangsstoffe für die Herstellung von Kunststoffen, Formmassen, Profilen, Lacken, Schmierölen, Klebern, Textilhilfsmitteln, Weichmacher etc. So werden die leicht polymerisierbaren Itaconsäurediester in der Kunststoffindustrie, z.B. in Verbindung mit Methacrylaten und Styrol, eingesetzt. Itaconsäurediester höherer Alkohole dienen als Spezialweichmacher für Kunstharze auf Polyvinylchloridbasis oder für Celluloseacetat und Ethylcellulose.

Die folgenden Beispiele dienen zur weiteren Erläuterung der Erfindung:

Beispiel 1

Eine Lösung von 0,3 g Essigsäure, 0,337 g Dimethylamin und 3,0 g Paraformaldehyd in 20 g Methanol wird auf 60 °C erwärmt. Unter Rühren wird eine Lösung von 11,6 g 2-Formylpropionsäuremethylester in 20 g Methanol innerhalb von 5 Minuten zugetropft. Anschließend wird 7 Stunden unter Rückfluß gekocht. Die quantitative gaschromatographische Analyse der Lösung zeigt, daß sich Methacrylsäuremethylester in einer Ausbeute von 86,6 % gebildet hat.

Beispiel 2

3,2 g Methanol, 0,328 g einer 36,5 %igen wäßrigen Formaldehydlösung, 0,0135 g Dimethylamin, 0,012 g Essigsäure und 0,464 g 2-Formylpropionsäuremethylester werden in einem druckfesten Glasrohr 15 Stunden auf 70 °C erhitzt. Durch quantitative gaschromatographische Analyse wurde die Ausbeute an Methacrylsäuremethylester zu 99 % bestimmt.

Beispiel 3

160 g Methanol, 0,842 g Dimethylamin, 0,75 g Essigsäure, 20,5 g einer 36,5 %igen wäßrigen Formaldehydlösung und 29,0 g 2-Formylpropionsäuremethylester werden in einem druckfesten Glasautoklaven 16 Stunden bei 70 °C gerührt. Die gaschromatographisch bestimmte Ausbeute an Methacrylsäuremethylester liegt bei 93,8 % der Theorie. Durch Destillation nach in der Literatur bekannten Verfahren kann Methacrylsäuremethylester abgetrennt und rein isoliert werden.

Beispiel 4

Ein Gemisch aus 64 g Methanol, 0,34 g Dimethylamin, 0,3 g Essigsäure, 8,2 g einer 36,5 %igen wäßrigen Formaldehydlösung und 13,0 g 2-Formylbuttersäuremethylester wird 69 Stunden bei 65 °C gerührt und gaschromatographisch analysiert. Die Ausbeute an 2-Ethylacrylsäuremethylester liegt bei 82 %

der Theorie.

Beispiel 5

Ein Gemisch aus 50 g Methanol, 0,34 g Dimethylamin, 0,3 g Essigsäure, 8,2 g einer 36,5 %igen wäßrigen Formaldehydlösung und 14,4 g 2-Formylvaleriansäuremethylester wird 26 Stunden bei 65° C gerührt und gaschromatographisch analysiert. Die Ausbeute an 2-(n-Propyl)acrylsäuremethylester liegt bei 72 % der Theorie, 27 % Ausgangsmaterial sind noch vorhanden.

Beispiel 6

Ein Gemisch aus 20 g Methanol, 0,71 g Dimethylamin, 0,15 g Essigsäure, 4,2 g einer 37 %igen wäßrigen Formaldehydlösung und 13,0 g 2-Formylprop-4-ensäuremethylester wird 26 Stunden bei 65° C gerührt und gaschromatographisch analysiert. Die Ausbeute an 2-Allylacrylsäuremethylester liegt bei 7,5 % der Theorie, 72 % Ausgangsmaterial sind noch vorhanden.

Beispiel 7

Zu einer Lösung aus 0,34 g Dimethylamin und 0,3 g Essigsäure in 40 g Methanol werden 3,0 g Paraformaldehyd und 17,8 g 2-Formyl-2-phenylessigsäuremethylester gegeben. Anschließend wird 8 Stunden unter Rückfluß gekocht. Die Ausbeute an 2-Phenylacrylsäuremethylester beträgt nach Destillation (100° C/0,3 mbar) 78 % der Theorie.

Beispiel 8

Zu einer Lösung von 9,0 g Oxalsäure und 14,6 g Diethylamin in 50 g Wasser werden 20 g einer 30 %igen Formaldehydlösung und 34,8 g 2-Formylbernsteinsäuredimethylester gegeben. Anschließend wird 4 Stunden auf 80° C erhitzt. Die abgeschiedene organische Phase enthält Itaconsäuredimethylester in einer Ausbeute von 38 %.

Beispiel 9

Zu einer Lösung von 14,8 g Propionsäure und 14,8 g Diethylamin in 160 g Methanol werden 6,0 g Paraformaldehyd und 34,8 g 2-Formylbernsteinsäuredimethylester gegeben. Das Reaktionsgemisch wird 5 Stunden bei 50° C gerührt und anschließend quantitativ gaschromatographisch analysiert. Die Ausbeute an Itaconsäuredimethylester beträgt 85 % der Theorie.

Beispiel 10

Analog Beispiel 8 werden 0,75 g Oxalsäure, 4,2 g Diethanolamin, 80 g Methanol, 6,0 g Paraformaldehyd und 34,8 g 2-Formylbernsteinsäuredimethylester eingesetzt. Nach 12 Stunden bei 50° C wird gaschromatographisch analysiert: Die Ausbeute an Itaconsäuredimethylester beträgt 65,4 % der Theorie.

Beispiel 11

Analog Beispiel 8 werden 0,3 g Essigsäure, 0,675 g Dimethylamin, 40 g Methanol, 3,0 g Paraformaldehyd und 17,4 g 2-Formylbernsteinsäuredimethylester eingesetzt. Nach 7 Stunden bei 50° C wird gaschromatographisch analysiert: Die Ausbeute an Itaconsäuredimethylester beträgt 91,4 % der Theorie.

Beispiel 12

Analog Beispiel 8 werden 0,3 g Essigsäure, 0,337 g Dimethylamin, 40 g Methanol, 3,0 g Paraformaldehyd und 17,4 g 2-Formylbernsteinsäuredimethylester eingesetzt. Nach 7 Stunden bei 50° C wird gaschromatographisch analysiert: Die Ausbeute an Itaconsäuredimethylester beträgt 99,0 % der Theorie.

Beispiel 13

Zu einer Lösung von 6,0 g Essigsäure und 6,75 g Dimethylamin in 800 g Methanol werden 60 g

Paraformaldehyd gegeben und anschließend innerhalb von 20 min 348 g 2-Formylbernsteinsäuredimethylester zugetropft. Man rührt 7 Stunden bei 50° C, läßt abkühlen und destilliert Methanol und Methylformiat ab. Der Rückstand wird in Essigsäureethylester aufgenommen, mit 100 g 10 %iger Schwefelsäure gewaschen und destilliert. Man erhält 305 g isomerenfreien Itaconsäuredimethylester vom Siedepunkt 42-43° C bei 0,3 bis 0,4 mbar entsprechend einer Ausbeute von 96,4 % der Theorie.

Beispiel 14

Ein Gemisch aus 20 g Ethanol, 0,337 g Dimethylamin, 0,3 g Essigsäure und 3 g Paraformaldehyd wird unter Rühren auf 50° C erwärmt und mit einer Lösung von 17,4 g 2-Formylbernsteinsäuredimethylester in 20 g Ethanol vereinigt. Man kocht 6 Stunden unter Rückfluß und analysiert gaschromatographisch. Die Ausbeute an Itaconsäuredimethylester beträgt 91 % der Theorie.

Beispiel 15

Ein Gemisch aus 40 g Methanol, 0,337 g Dimethylamin, 0,3 g Essigsäure, 3 g Paraformaldehyd und 25,8 g 2-Formylbernsteinsäuredi-n-butylester wird 4 Stunden unter Rückfluß gerührt. Die gaschromatographische Analyse zeigt eine Ausbeute an Itaconsäuredi-n-butylester von 98 % der Theorie; Umesterungen zum Methylester ließen sich nicht nachweisen.

Beispiel 16

Ein Gemisch aus 40 g Butanol, 0,337 g Dimethylamin, 0,3 g Essigsäure, 3 g Paraformaldehyd und 25,8 g 2-Formylbernsteinsäuredi-n-butylester wird 4 Stunden bei 65° C gerührt und gaschromatographisch analysiert. Die Ausbeute an Itaconsäuredi-n-butylester liegt bei 82 % der Theorie.

Beispiel 17

Ein Gemisch aus 165 g Methanol, 1,39 g Dimethylamin, 1,24 g Essigsäure, 12,38 g Paraformaldehyd und 64 g 2-Formylbutyrolacton wird 22 Stunden bei 65° C gerührt. Methanol wird abdestilliert und der Rückstand gaschromatographisch analysiert. Die Ausbeute an 2-Methylenbutyrolacton liegt bei 72 % der Theorie.

Beispiel 18

Ein Gemisch aus 40 g Methanol, 0,34 g Dimethylamin, 0,3 g Essigsäure, 8,2 g einer 37 %igen wäßrigen Formaldehydlösung und 11,2 g 2-Formylcyclopentanon wird 3 Stunden bei 65° C gerührt und gaschromatographisch analysiert. Die Ausbeute an 2-Methylencyclopentanon liegt bei 56 % der Theorie.

Beispiel 19

Ein Gemisch aus 40 g Methanol, 0,34 g Dimethylamin, 0,3 g Essigsäure, 8,2 g einer 37 %igen wäßrigen Formaldehydlösung und 13,8 g 2-Formylpropionitril wird 23 Stunden bei 65° C gerührt und gaschromatographisch analysiert. Die Ausbeute an Methylacrylnitril liegt bei 32 % der Theorie, 67 % Ausgangsmaterial sind noch vorhanden.

Beispiel 20

Ein Gemisch aus 40 g Methanol, 0,34 g Dimethylamin, 0,3 g Essigsäure, 8,2 g einer 36,5 %igen wäßrigen Formaldehydlösung und 10,8 g 2-Formylbernsteinsäuredinitril wird 2 Stunden bei 65° C gerührt und gaschromatographisch analysiert. Die Ausbeute an Itaconsäuredinitril liegt bei 69 % der Theorie.

Beispiel 21

Ein Gemisch aus 20 g Methanol, 0,17 g Dimethylamin, 0,15 g Essigsäure, 4,2 g einer 37 %igen wäßrigen Formaldehydlösung und 9,25 g 2-Formyl-2-(2-pyrrolidon-1-yl)essigsäuremethylester wird 2 Stunden unter Rückfluß gekocht und gaschromatographisch analysiert. Die Ausbeute an 2-(2-Pyrrolidon-1-yl)-acrylsäuremethylester liegt bei 20 % der Theorie.

Beispiel 22

Ein Gemisch aus 40 g Methanol, 0,34 g Dimethylamin, 0,3 g Essigsäure, 8,2 g einer 36,5 %igen wäßrigen Formaldehydlösung und 15,9 g 2,N-Diformylaminoessigsäureethylester wird 20 Stunden bei 65° C gerührt und gaschromatographisch analysiert. Die Ausbeute an 2-(N-Formylamino)acrylsäureethylester liegt bei 20 % der Theorie.

## Patentansprüche

**1.** Verfahren zur Herstellung von 1,1-disubstituierten Ethylenverbindungen der allgemeinen Formel I

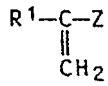

I,

in der
Z $COOR^2$, CN oder $COR^3$,
$R^1$ einen aliphatischen, cycloaliphatischen, araliphatischen, aromatischen oder heterocyclischen Rest, der gegebenenfalls durch unter den Reaktionsbedingungen inerte funktionelle Gruppen weitersubstituiert sein kann,
$R^2$ einen aliphatischen, cycloaliphatischen oder araliphatischen Rest mit 1 bis 15 Kohlenstoffatomen und
$R^3$ einen aliphatischen, cycloaliphatischen oder araliphatischen Rest mit 1 bis 15 Kohlenstoffatomen, der durch unter den Reaktionsbedingungen inerte Gruppen substituiert sein kann,

bedeuten, wobei $R^1$ mit $R^2$ bzw. $R^1$ mit $R^3$ aber auch eine Alkylenkette mit 2 bis 10 Kohlenstoffatomen bilden kann, die durch unter den Reaktionsbedingungen inerte Gruppen substituiert sein kann, aus Formylverbindungen der allgemeinen Formel II

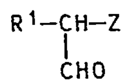

II,

in der Z, $R^1$, $R^2$ und $R^3$ die oben genannten Bedeutungen besitzen,
dadurch gekennzeichnet, daß man die Umsetzung in Gegenwart eines sekundären Amins, einer Protonensäure und von Formaldehyd oder Paraformaldehyd und
a) einem $C_1$-$C_{12}$-Alkanul oder
b) einem Gemisch aus einem $C_1$-$C_{12}$-Alkanol und Wasser oder
c) mit Wasser
bei Temperaturen von 0 bis 200° C vornimmt.

**2.** Verfahren nach Anspruch 1, dadurch gekennzeichnet, daß man die Umsetzung in Gegenwart von 0,5 bis 2 mol Formaldehyd pro Mol Formylverbindung II vornimmt.

**3.** Verfahren nach den Ansprüchen 1 oder 2, dadurch gekennzeichnet, daß man die Umsetzung in einem $C_1$- bis $C_8$-Alkanol vornimmt.

**4.** Verfahren nach den Ansprüchen 1 bis 3, dadurch gekennzeichnet, daß man die Umsetzung in Methanol vornimmt.

**5.** Verfahren nach den Ansprüchen 1 bis 4, dadurch gekennzeichnet, daß man die Umsetzung in 1 bis 50 mol Alkanol pro Mol Formylverbindung II vornimmt.

**6.** Verfahren nach den Ansprüchen 1 oder 2, dadurch gekennzeichnet, daß man die Umsetzung in Gegenwart einer aliphatischen Monocarbonsäure mit 2 bis 5 Kohlenstoffatomen als Protonensäure vornimmt.

7. Verfahren nach den Ansprüchen 1, 2 oder 6, dadurch gekennzeichnet, daß man die Umsetzung in Gegenwart von 0,005 bis 1,5 mol einer Protonensäure pro Mol Formylverbindung II vornimmt.

8. Verfahren nach den Ansprüchen 1, 2, 6 oder 7, dadurch gekennzeichnet, daß man die Umsetzung in Gegenwart von 0,005 bis 1,5 mol eines $C_2$-$C_{10}$-Dialkylamins pro Mol Formylverbindung II vornimmt.

**Claims**

1. A process for the preparation of a 1,1-disubstituted ethylene compound of the formula I

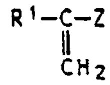

I

where Z is $COOR^2$, CN or $COR^3$, $R^1$ is an aliphatic, cycloaliphatic, araliphatic, aromatic or heterocyclic radical which may be further substituted by functional groups which are inert under the reaction conditions, $R^2$ is an aliphatic, cycloaliphatic or araliphatic radical of 1 to 15 carbon atoms and $R^3$ is an aliphatic, cycloaliphatic or araliphatic radical of 1 to 15 carbon atoms which may be substituted by groups which are inert under the reaction conditions, and $R^1$ together with $R^2$ or $R^1$ together with $R^3$ may furthermore form an alkylene chain of 2 to 10 carbon atoms which may be substituted by groups which are inert under the reaction conditions, from a formyl compound of the formula II

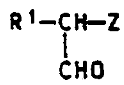

II

where Z, $R^1$, $R^2$ and $R^3$ have the above meanings, wherein the reaction is carried out in the presence of a secondary amine, a protic acid and of formaldehyde or paraformaldehyde and
a) a $C_1$-$C_{12}$-alkanol or
b) a mixture of a $C_1$-$C_{12}$-alkanol and water or
c) with water
at from 0 to 200 °C.

2. A process as claimed in claim 1, wherein the reaction is carried out in the presence of from 0.5 to 2 moles of formaldehyde per mole of formyl compound II.

3. A process as claimed in claim 1 or 2, wherein the reaction is carried out in a $C_1$-$C_8$-alkanol.

4. A process as claimed in any of claims 1 to 3, wherein the reaction is carried out in methanol.

5. A process as claimed in any of claims 1 to 4, wherein the reaction is carried out in from 1 to 50 moles of alkanol per mole of formyl compound II.

6. A process as claimed in claim 1 or 2, wherein the reaction is carried out in the presence of an aliphatic monocarboxylic acid of 2 to 5 carbon atoms as the protic acid.

7. A process as claimed in any one of claims 1, 2 or 6, wherein the reaction is carried out in the presence of from 0.005 to 1.5 moles of a protic acid per mole of formyl compound II.

8. A process as claimed in any one of claims 1, 2, 6 or 7, wherein the reaction is carried out in the presence of from 0.005 to 1.5 moles of a $C_2$-$C_{10}$-dialkylamine per mole of formyl compound II.

**Revendications**

**1.** Procédé de préparation de dérivés de l'éthylène 1,1-disubstitués de formule générale I

$$\begin{array}{c} R^1 - C - Z \\ \quad\;\; \| \\ \quad\;\; CH_2 \end{array} \qquad\qquad I,$$

dans laquelle

- Z représente $COOR^2$, CN ou $COR^3$,
- $R^1$ représente un reste aliphatique, cycloaliphatique, araliphatique, aromatique ou hétérocyclique qui peut éventuellement être substitué par des groupements fonctionnels inertes dans les conditions de réaction,
- $R^2$ représente un reste aliphatique, cycloaliphatique ou araliphatique ayant de 1 à 15 atomes de carbone, et
- $R^3$ représente un reste aliphatique, cycloaliphatique ou araliphatique ayant de 1 à 15 atomes de carbone qui peut éventuellement être substitué par des groupements inertes dans les conditions de réaction,

$R^1$ avec $R^2$ ou $R^1$ avec $R^3$ pouvant aussi former une chaîne alkylène de 2 à 10 atomes de carbone qui peut être substituée par des groupements inertes dans les conditions de réaction,
à partir de dérivés formylés de formule générale II

$$\begin{array}{c} R^1 - CH - Z \\ \quad\;\; | \\ \quad\;\; CHO \end{array} \qquad\qquad II$$

dans laquelle Z, $R^1$, $R^2$ et $R^3$ sont tels que définis précédemment,
caractérisé en ce qu'on mène la réaction à des températures de 0 à 200 °C en présence d'une amine secondaire, d'un acide protonique et de formaldéhyde ou de paraformaldéhyde et

a) d'un alcool en $C_1$-$c_{12}$ ou
b) d'un mélange d'un alcool en $C_1$-$C_{12}$ et d'eau ou
c) avec de l'eau.

**2.** Procédé selon la revendication 1, caractérisé en ce qu'on mène la réaction en présence de 0,5 à 2 moles de formaldéhyde par mole de composé formylé II.

**3.** Procédé selon la revendication 1 ou 2, caractérisé en ce qu'on mène la réaction dans un alcanol en $C_1$-$C_8$.

**4.** Procédé selon l'une quelconque des revendications 1 à 3, caractérisé en ce qu'on mène la réaction dans le méthanol.

**5.** Procédé selon l'une quelconque des revendications 1 à 4, caractérisé en ce qu'on mène la réaction dans 1 à 50 moles d'alcanol par mole de composé formylé II.

**6.** Procédé selon l'une ou l'autre des revendications 1 et 2, caractérisé en ce qu'on mène la réaction en présence d'un acide monocarboxylique aliphatique ayant de 2 à 5 atomes de carbone comme acide protonique.

**7.** Procédé selon l'une quelconque des revendications 1, 2 et 6, caractérisé en ce qu'on mène la réaction en présence de 0,005 à 1,5 mole d'un acide protonique par mole de composé formylé II.

**8.** Procédé selon l'une quelconque des revendications 1, 2, 6 et 7, caractérisé en ce qu'on mène la réaction en présence de 0,005 à 1,5 mole d'une di(alkyl en $C_2$-$C_{10}$)amine par mole de dérivé formylé II.